# EUROPEAN PATENT APPLICATION

(11) **EP 4 736 742 A1**
(43) Date of publication of application: **06.05.2026**
(21) Application number: 24210096.4
(22) Date of filing: 31.10.2024
(51) Int. Cl.: A61B 5/00

(54) **SENSOR SUPPORT STRAP, SYSTEM AND METHODS OF USE AND MANUFACTURE OF THE SENSOR SUPPORT STRAP**

(71) Applicant: Koninklijke Philips N.V., 5656 AG Eindhoven (NL)
(72) Inventor: VAN VELDHUIZEN, Gijsbert Hendrik, Eindhoven (NL)
(74) Representative: Philips Intellectual Property & Standards

(57) **Abstract**

Provided is a sensor support strap (10) for allowing a subject to wear a sensor arrangement supported by the strap. The sensor support strap has a strap member securable to a body part of the subject. The sensor support strap has a flap (12), a main portion (14) and a hinging portion (16) provided between the flap and the main portion. The sensor support strap has an edge (34) which at least partly defines an opening of the strap member in which the body part is receivable when the strap member is secured to the body part of the subject such that a flap region (18) of the edge is contactable by the body part. The flap is at least partly delimited by the flap region and a flap side edge (32). The flap side edge extends from the flap region to reach the hinging portion so as to cause the strap member to preferentially hinge at the hinging portion. Additionally provided is a system comprising the sensor support strap and a sensor arrangement for being supported by the strap. Further provided is a method of use of the sensor support strap and a method of manufacturing the sensor support strap.

## Description

### FIELD OF THE INVENTION

The invention relates to a sensor support strap for allowing a subject to wear a sensor arrangement supported by the strap. The invention additionally concerns a system comprising the sensor support strap and a sensor arrangement for being supported by the strap.

The invention also relates to use of the sensor support strap for allowing a subject to wear a sensor arrangement supported by the strap.

The invention further provides a method of manufacturing the sensor support strap.

### BACKGROUND OF THE INVENTION

Various sensor support straps exist for allowing a subject to wear sensors on their body, such as vital signs sensors. Often, a subject will be required to wear such a strap for a prolonged period of time for monitoring purposes. Accordingly, the comfort of the user whilst wearing the strap is important.

A common problem with such sensor support straps is that they can leave pressure marks on the subject's body. This can be the case when the strap tension is too high, or simply when the strap has been worn for a prolonged period of time. For example, in the case of baby monitoring systems which include a strap for securing around the baby's foot and/or ankle, pressure marks are often observed in the region where the top of the foot, e.g. the instep, meets the bottom of the shin.

One approach for improving user comfort would be to simply design the strap such that the edge is located away from such a pressure mark prone area, thereby avoiding contact between the edge of the strap and this area.

For example, in the case of a strap for securing around a foot and ankle, the strap may be designed such that the relevant edge is intended to sit further down the foot, e.g. further down the instep, away from the area between the instep and the shin. However, this may be undesirable since the user will often attempt to align said edge with this area regardless of the intended positioning of the strap, resulting in no improvement in comfort, and incorrect sensor placement.

### SUMMARY OF THE INVENTION

It would be desirable to provide a sensor support strap for allowing a subject to wear a sensor arrangement supported by the strap which provides enhanced comfort for the subject.

The invention is defined by the claims.

According to examples in accordance with a first aspect of the invention, there is provided a sensor support strap for allowing a subject to wear a sensor arrangement supported by the strap, the sensor support strap comprising: a strap member securable to a body part of the subject, the strap member comprising: a flap, a main portion, a hinging portion provided between the flap and the main portion, and an edge which at least partly defines an opening of the strap member in which the body part is receivable when the strap member is secured to the body part of the subject such that a flap region of the edge is contactable by the body part, wherein the flap is at least partly delimited by the flap region and a flap side edge, the flap side edge extending from the flap region to reach the hinging portion so as to cause the strap member to preferentially hinge at the hinging portion.

In this way, a pressure exerted by at least the flap region of the edge on the subject may be reduced. This is because the flap may be able to hinge, in other words fold, to accommodate movement and/or a shape of the subject, e.g. a shape of the body part and/or movement of the body part, e.g. a bending area of the body part. This may enhance the comfort of the subject and reduce the likelihood of the subject experiencing red marks as a result of the pressure exerted by the edge.

The hinging portion can be provided in any suitable manner. In some embodiments, the hinging portion is provided by a line of weakness at which the strap member preferentially folds.

The line of weakness can be formed, for example, by the strap member being compressed, e.g. by a folding process in which the flap is folded relative to the main portion, along a line defining a boundary between the flap and the main portion.

In other embodiments, the hinging portion is provided by a strip of material which separates the flap from the main portion, which strip of material is more flexible than the flap and the main portion so that the strip of material flexes, rather than the flap and the main portion, during hinging of the strap member at the hinging portion.

The body part may comprise a moveable joint of the subject. The edge may, for example, be contactable by a bending area of the moveable joint.

In some embodiments, the strap member comprises at least one relief region separating the flap side edge from an adjacent portion of the strap member, the at least one relief region being configured to enlarge when the flap preferentially hinges at the hinging portion.

An enlargeable at least one relief region may enable the opening to enlarge to accommodate movement and/or the shape of the subject, in addition to the flap being able to hinge to accommodate movement and/or the shape of the subject. Accordingly, inclusion of an at least one relief region may further enhance the comfort of the subject and reduce the likelihood of the subject experiencing red marks as a result of the pressure exerted by at least the flap region of the edge.

Enlargement of the at least one relief region may comprise an increase in a distance from the flap side edge to the adjacent portion. In this way, enlargement of the at least one relief region may result in enlargement of the opening.

In some embodiments, the sensor support strap may not include such a relief region. For example, in embodiments in which the flap, e.g. the flap side edge, protrudes directly from the edge, the flap may not be separated from an adjacent portion by any relief region, but may still be able to hinge at the hinging portion. However, in such embodiments, the opening may not be able to enlarge in the same way as when the relief region(s) is/are provided. Accordingly, the inclusion of the at least one relief region may provide enhanced comfort for the subject compared to when such a relief region is not included.

In some embodiments, the at least one relief region comprises a split in the strap member. A split may provide an effective relief region, whilst also being straightforward to manufacture. For example, the split may be provided by cutting the strap member.

In some embodiments, the at least one relief region comprises a flexible gusset connecting the flap to the adjacent portion.

A flexible gusset may prevent detritus from falling into the sensor support strap, e.g. between the strap and the body part, whilst still allowing the flap to hinge at the hinging portion. Inclusion of a flexible gusset may reduce the likelihood of flap side edge and/or the adjacent portion, e.g. an edge of the adjacent portion, pinching the subject's skin.

In some embodiments, the flap comprises a laminate having two or more layers, the flexible gusset having fewer layers than the laminate such that the stiffness of the flexible gusset is lower than the stiffness of the flap.

This may present a particularly convenient way of providing a flexible gusset which is straightforward to manufacture. For example, the flexible gusset may be provided by removing or omitting one or more layers of the laminate of the flap at the location(s) of the relief region(s), such that the remaining layers of the laminate material form the flexible gusset. In this way, the flexible gusset may be integral to the flap and the adjacent portion, and there may be no need for additional joining processes to attach the flexible gusset to the flap and the adjacent portion.

In some embodiments, at least one of the relief region(s) and a sensor location of the main strap are visible to the subject when the sensor support strap is being donned by the subject.

In this way, the relief region(s) and/or the sensor location may be used as alignment feature(s) for enabling the sensor support strap to be positioned correctly on the subject.

In some embodiments, the sensor support strap comprises an elongate strap element for wrapping at least partly around the body part, with a strap element relief region, of the at least one relief region, separating part of the elongate strap element from the flap. Thus, the strap may be effectively secured to the body part whilst the comfort of the subject may be enhanced.

A single flap may, for example, be provided extending away from the elongate strap element. This may be an advantageous configuration of the sensor support strap for use on certain body parts, such as body parts with less curvature.

In some embodiments, the sensor support strap comprises a further elongate strap element joinable to the elongate strap element, with the further elongate strap element having a shorter length than the elongate strap element.

In such embodiments, the strap element relief region may be located closer to the elongate strap element than to the further elongate strap element. Thus, ease of securing the strap to the subject may be enhanced. Positioning the strap element relief region at the side of the longer strap, e.g. the elongate strap, may improve the stiffness of the shorter strap, e.g. the further elongate strap, resulting in improved handling of the shorter strap.

In some embodiments, the sensor support strap comprises a further flap and a second hinging portion for allowing hinging of the further flap to enlarge the opening.

By providing a further flap, e.g. a second flap, the sensor support strap may be able to more effectively accommodate movement and/or the shape of the subject, reducing discomfort for the user. This may be an advantageous configuration of the sensor support strap for use on certain body parts, such as smaller, more curved body parts.

In some embodiments, the further flap defines the adjacent portion, with the further flap being separated from the flap by one of said at least one relief region. In other words, two flaps, namely the flap and the further flap, may be straightforwardly separated from each other by one relief region.

In some embodiments, the sensor support strap is configured for use on a foot of the subject. For example, the body part may comprise at least a portion of the foot, with the portion comprising at least part of the ankle of the foot. In such embodiments, the bending area may comprise part of the ankle between the subject's instep and the subject's shin.

The subject may be an infant, such that the sensor support strap is configured for use on a foot of an infant, in other words a baby's foot.

In embodiments in which the sensor support strap is configured for use on a foot, the relief region and the sensor location may be alignable during use along a notional line extending from the subject's ankle towards one or more toes on the foot.

In some embodiments, the sensor support strap comprises a further edge, with the further edge comprising a notch which is alignable with one or more toes of the foot during use. The notch may be used for alignment of the sensor support strap on the foot.

In at least some embodiments, the relief region, the notch and a sensor location of the sensor support strap may be aligned along a (further) notional line extending from the relief region to the notch. This (further) notional line may be the same as or different from the above-mentioned notional line extending from the subject's ankle towards one or more toes on the foot.

According to examples in accordance with a second aspect of the invention, there is provided a sensor support strap system comprising a sensor support strap of any of the embodiments described in relation to the first aspect of the invention, and a sensor arrangement suitable for being supported by the strap.

According to examples in accordance with a third aspect of the invention, there is provided a use of a sensor support strap, the use comprising allowing a subject to wear a sensor arrangement supported by the strap, the sensor support strap comprising: a strap member securable to a body part of the subject, the strap member comprising: a flap; a main portion; and a hinging portion provided between the flap and the main portion; and an edge which at least partly defines an opening of the strap member in which the body part is receivable when the strap member is secured to the body part of the subject such that a flap region of the edge is contactable by the body part, wherein the flap is at least partly delimited by the flap region and a flap side edge, the flap side edge extending from the flap region to reach the hinging portion so as to cause the strap member to preferentially hinge at the hinging portion.

In some embodiments, the use comprises allowing the subject to wear the sensor support strap comprising the strap member, wherein the strap member comprises at least one relief region separating the flap side edge from an adjacent portion of the strap member, with the at least one relief region being configured to enlarge when the flap preferentially hinges at the hinging portion.

In some embodiments, the use comprises allowing the subject to wear the sensor support strap comprising the at least one relief region, wherein the at least one relief region comprises a split in the strap member.

Alternatively or additionally, the at least one relief region comprises a flexible gusset connecting the flap to the adjacent portion.

The use of the sensor support strap of the third aspect may comprise use of the sensor support strap of any of the embodiments disclosed herein in relation to the first aspect of the invention.

The body part may comprise a moveable joint of the subject. The edge may, for example, be contactable by a bending area of the moveable joint.

The sensor support strap may be configured for use on a foot of the subject. That is, the body part may comprise at least a portion of the foot, with the portion comprising at least part of the ankle of the foot. In such embodiments, the bending area may comprise part of the ankle between the subject's instep and the subject's shin.

The subject may be an infant and the body part may be a foot. In other words the sensor support strap may be configured for use on a foot of an infant, such as a baby's foot.

According to examples in accordance with a fourth aspect of the invention, there is provided a method of manufacturing a sensor support strap for allowing a subject to wear a sensor arrangement supported by the strap, the sensor support strap comprising: a strap member securable to a body part of the subject, the strap member comprising: a flap; a main portion; a hinging portion provided between the flap and the main portion; and an edge which at least partly defines an opening of the strap member in which the body part is receivable when the strap member is secured to the body part of the subject such that a flap region of the edge is contactable by the body part, wherein the flap is at least partly delimited by the flap region and a flap side edge, wherein the method comprises providing the strap member with a hinging portion, the strap member comprising a flap side edge extending from the flap region to reach the hinging portion so as to cause the strap member to preferentially hinge at the hinging portion.

The method step of providing the strap member with the hinging portion can be implemented in any suitable manner. In some embodiments, the method step of providing the strap member with the hinging portion comprises providing a line of weakness, extending along a line defining a boundary between the flap from the main portion, at which the strap member preferentially folds.

The line of weakness can be formed, for example, by compressing the strap member, e.g. by folding the flap relative to the main portion, along the line defining the boundary between the flap and the main portion.

In other embodiments, providing the hinging portion comprises providing a strip of material to separate the flap from the main portion, which strip of material is more flexible than the flap and the main portion so that the strip of material flexes, rather than the flap and the main portion, during hinging of the strap member at the hinging portion.

In some embodiments, the method further comprises providing the strap member with at least one relief region, wherein the at least one relief region separates the flap side edge from an adjacent portion of the strap member, the at least one relief region being configured to enlarge when the flap preferentially hinges at the hinging portion.

In some embodiments, providing the strap member with at least one relief region comprises providing a split in the strap member.

Alternatively or additionally, providing the strap member with at least one relief region comprises providing a flexible gusset connecting the flap to the adjacent portion.

The method of the fourth aspect may comprise providing the sensor support strap, e.g. the strap member, of any of the embodiments disclosed herein in relation to the first aspect of the invention.

The body part may comprise a moveable joint of the subject. The edge may, for example, be contactable by a bending area of the moveable joint.

The sensor support strap may be configured for use on a foot of the subject. That is, the body part may comprise at least a portion of the foot, with the portion comprising at least part of the ankle of the foot. In such embodiments, the bending area may comprise part of the ankle between the subject's instep and the subject's shin.

The subject may be an infant and the body part may be a foot. In other words the sensor support strap may be configured for use on a foot of an infant, such as a baby's foot.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a sensor support strap according to an example;
FIG. 2 provides a sensor support strap according to an example;
FIG. 3 provides a sensor support strap according to an example;
FIG. 4 provides a sensor support strap according to an example;
FIG. 5 provides a sensor support strap according to an example;
FIG. 6 provides a sensor support strap according to an example;
FIG. 7 provides a sensor support strap according to an example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a sensor support strap for allowing a subject to wear a sensor arrangement supported by the strap. The sensor support strap has a strap member securable to a body part of the subject. The sensor support strap has a flap, a main portion and a hinging portion provided between the flap and the main portion. The sensor support strap has an edge which at least partly defines an opening of the strap member in which the body part is receivable when the strap member is secured to the body part of the subject such that a flap region of the edge is contactable by the body part. The flap is at least partly delimited by the flap region and a flap side edge. The flap side edge extends from the flap region to reach the hinging portion so as to cause the strap member to preferentially hinge at the hinging portion. Additionally provided is a system comprising the sensor support strap and a sensor arrangement for being supported by the strap. Further provided is a method of use of the sensor support strap and a method of manufacturing the sensor support strap.

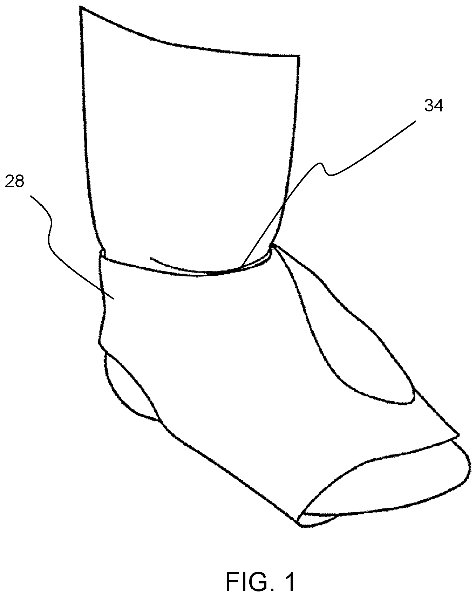

FIGs. 1 to 4 show examples of sensor support straps for comparison with the present disclosure.

The sensor support straps shown in FIGs. 1 to 4 are configured to allow a subject to wear a sensor on their foot, such as at sensor locations A, B or C. However, it is noted that the ideas discussed in relation to these examples are equally applicable to sensor support straps configured for use on other parts of the body.

The sensor support straps of FIGs. 1 to 4 include a pair of elongate strap members 28, 30 which may be secured to each other to secure the sensor support strap around the ankle. Also shown in each of FIGs. 1 to 4 are a pair of additional straps which may be secured to each other below the sole of the foot to further secure the sensor support strap to the foot.

When the elongate strap members 28, 30 are secured around the subject's ankle, an opening of the strap member is defined by an edge 34. Accordingly, the edge 34 may exert a pressure on the subject (see, for example, FIG. 1). This may result in patient discomfort and marks on the skin. This discomfort may, for example, be exacerbated by overtightening the strap, movement of the body part, or by prolonged periods of wearing the strap.

The above-described issue of patient discomfort may exist regardless of the particular securement method employed by the sensor support strap. That is, the issue may exist for any sensor support strap which includes such an edge 34 which is contactable by a body part, such a sensor support strap configured as a sock to be slipped over the subject's foot which includes the edge 34.

Whilst the sensor support straps of FIGs. 1 to 4 are configured to be secured around a subject's foot and ankle, such straps may equally be configured for use on another body part, such as a wrist, an elbow or a knee. In such configurations, the subject may experience discomfort in the manner described above as a result of such an edge 34 which contacts the body part, e.g. a bending area of the body part, such as the inside of a knee, the inside of an elbow, or the underside of a wrist.

One approach to reducing such discomfort would be to simply move the edge 34 away from the pressure mark prone area, thereby avoiding contact between the edge 34 and this area.

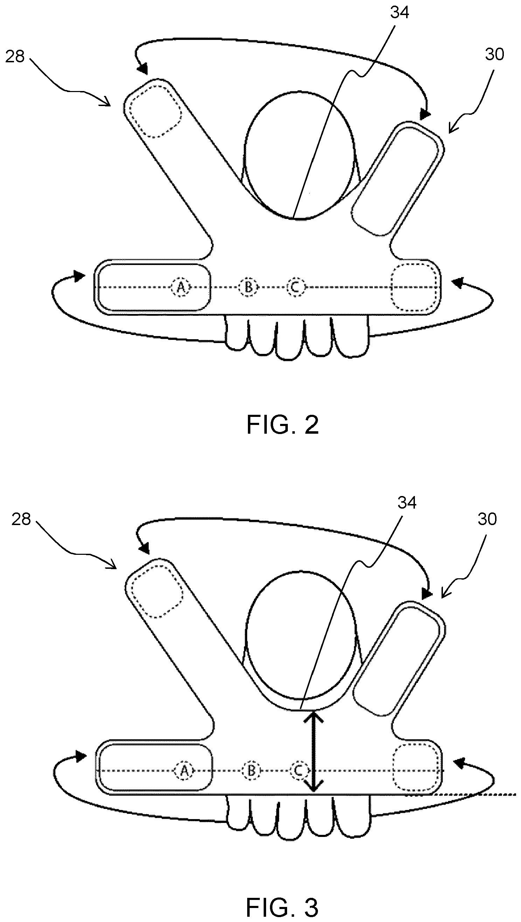

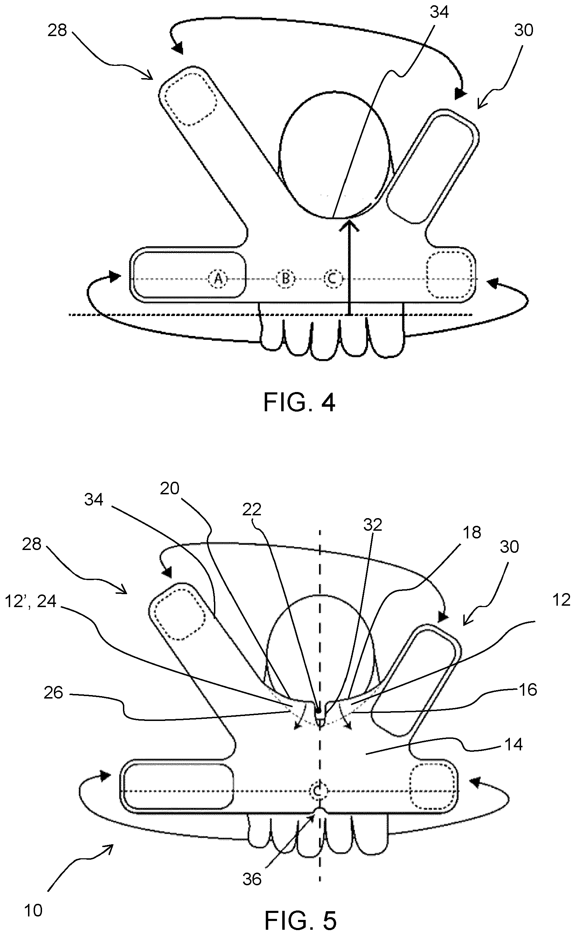

For example, in the case of a sensor support strap for use on a foot, the strap may be designed such that the relevant edge 34 is intended to sit further down the foot, e.g. further down the instep, away from the bending area of the ankle, as indicated by the double headed arrow on the sensor support strap of FIG. 3. However, this may be undesirable since the user will often attempt to align said edge 34 with this bending area regardless of the intended strap use, as illustrated by the single headed arrow on the sensor support strap 10 of FIG. 4, resulting in incorrect sensor positioning and no improvement in comfort. Moreover, such an edge 34 can in fact prove useful for achieving correct sensor positioning, by aligning the edge 34 with a feature of the body part, whether this is a foot, an ankle, or any other body part.

It would be desirable to provide a sensor support strap for allowing a subject to wear a sensor arrangement supported by the strap which provides enhanced comfort for the subject.

Referring to FIGs. 5 to 7, the present disclosure accordingly provides a sensor support strap 10 for allowing a subject to wear a sensor arrangement supported by the sensor support strap 10. The sensor support straps 10 of FIGs. 5 to 7 share some features with the comparative examples of FIGs. 1 to 4 but include additional features which provide enhanced comfort for the subject.

The sensor support strap 10, as shown in FIGs. 5 to 7, comprises a strap member securable to a body part of the subject. The strap member further comprises a flap 12, a main portion 14 and a hinging portion 16 (as indicated by a dotted line 16 in FIGs. 5 to 7) provided between the flap 12 and the main portion 14.

The sensor support strap 10 also includes an edge 34, which is similar to the edge 34 of FIGs. 1 to 4. The edge 34 at least partly defines an opening of the strap member in which the body part is receivable when the strap member is secured to the body part of the subject such that a flap region 18 of the edge 34 is contactable by the body part. For example, the flap region 18 of the edge 34 may be contactable by the body part where the top of the foot meets the bottom of the shin (see FIGs. 5 to 7).

The flap 12 is at least partly delimited by the flap region and a flap side edge 32 which extends from the flap region 18 of the edge 34 to reach the hinging portion 16 so as to cause the strap member to preferentially hinge at the hinging portion 16.

Accordingly, a pressure exerted by at least the flap region 18 of the edge 34 on the subject may be reduced, compared to the straps of FIGs. 1 to 4 which do not include the flap 12. This is because the flap 12 may be able to hinge, in other words fold, to accommodate movement and/or a shape of the subject, e.g. a shape of the body part and/or movement of the body part, e.g. a bending area of the body part. This may enhance the comfort of the subject and reduce the likelihood of the subject experiencing red marks as a result of the pressure exerted by the edge.

The hinging portion 16 can be provided in any suitable manner. In some embodiments, the hinging portion 16 is provided by a line of weakness at which the strap member preferentially folds.

The line of weakness can be formed, for example, by the strap member being compressed, e.g. by a folding process in which the flap 12 is folded relative to the main portion 14, along a line defining a boundary between the flap 12 and the main portion 14. For example, the strap member may have been compressed during manufacture of the strap 10.

In other embodiments, the hinging portion 16 is provided by a strip of material which separates the flap 12 from the main portion 14, which strip of material is more flexible than the flap 12 and the main portion 14 so that the strip of material flexes, rather than the flap 12 and the main portion 14, during hinging of the strap member at the hinging portion 16.

The body part may comprise a moveable joint of the subject. The edge 34 may, for example, be contactable by a bending area of the moveable joint.

In some embodiments, such as those of FIGs. 5 to 7, the sensor support strap 10 is configured for use on a foot of the subject. Accordingly, as explained above, the flap region 18 of the edge 34 may be contactable by the area where the foot meets the bottom of the shin. However, it is reiterated that the sensor support strap 10 may also be configured for use on any other body part, such as a wrist, an elbow or a knee. In such configurations, the same features and mechanisms as described in relation to FIGs. 5 to 7, e.g. the flap 12, may be employed to assist in reducing the pressure applied to the subject by the edge 34.

The sensor support strap may, for example, be made from a fabric material, such as a fabric which comprises elastomeric fibers.

In some embodiments, such as those shown in FIGs. 5 to 7, the strap member comprises at least one relief region 22 separating the flap side edge 32 from an adjacent portion 24 of the strap member, the at least one relief region 22 being configured to enlarge when the flap 12 preferentially hinges at the hinging portion 16.

An enlargeable at least one relief region 22 may enable the opening to enlarge to accommodate movement and/or the shape of the subject, in addition to the flap 12 being able to hinge to accommodate movement and/or the shape of the subject. Accordingly, inclusion of an at least one relief region 22 may further enhance the comfort of the subject and reduce the likelihood of the subject experiencing red marks as a result of the pressure exerted by at least the flap region 18 of the edge 34.

In some embodiments, the sensor support strap 10 may not include such a relief region 22. For example, in embodiments in which the flap, e.g. the flap side edge 32, protrudes directly from the edge 34, the flap 12 may not be separated from an adjacent portion 24 by any relief region 22, but may still be able to hinge at the hinging portion 16. However, in such embodiments, the opening may not be able to enlarge in the same way as when the relief region(s) 22 is/are provided. Accordingly, the inclusion of the at least one relief region 22 may provide enhanced comfort for the subject compared to when such a relief region is not included

In some embodiments, such as those of FIGs. 5 to 7, the at least one relief region 22 may be a split in the strap member.

For example, the split may be provided by simply cutting a slit in the strap member, e.g. a slit extending from the flap region 18 of the edge 34 so that a side of the split forms the flap side edge 32. Alternatively, and as shown in FIGs. 5 to 7, the split may be provided, e.g. cut, in the strap member such that there is a visible gap between the flap side edge 32 and the adjacent portion 24. Both designs may provide effective relief regions 22, whilst being straightforward to manufacture.

In some embodiments, the at least one relief region 22 may include a flexible gusset connecting the flap 12 to the adjacent portion 24. Although not shown in FIGs. 5 to 7, these non-limiting examples could include a flexible gusset which occupies the relief region 22, e.g. the area defined by the split as shown in FIGs. 5 to 7. The flexible gusset may, for example, occupy the entire area defined by the split, or only a portion of the area defined by the split.

A flexible gusset may prevent detritus from falling into the sensor support strap 10, e.g. between the strap 10 and the body part, whilst still allowing the flap 12 to hinge at the hinging portion 16.

Inclusion of a flexible gusset may reduce the likelihood of flap side edge and/or the adjacent portion, e.g. an edge of the adjacent portion, from pinching the subject's skin.

The flexible gusset may be made from a material, e.g. an elastomeric material, which is able to stretch when the relief region 22 enlarges. Alternatively or additionally, the flexible gusset may include excess material, e.g. the gusset may be pleated, such the pleats of the flexible gusset may fold and unfold when the relief region 22 enlarges or decreases in size.

One way in which the gusset could be provided is by first providing the split, and subsequently attaching the gusset to the strap member, e.g. to the flap side edge 32 and the adjacent portion 24. This may be a particularly straightforward method of providing the flexible gusset when the flap 12 is made of fabric, e.g. a fabric comprising elastomeric fibers. Alternatively, the flap 12, adjacent portion 24 and/or the main portion 14 may be reinforced using additional material, e.g. a stiffer material. In this way an area between the flap 12 and the adjacent portion 24, that being the flexible gusset, may be relatively more flexible than the flap 12 and the adjacent portion 24.

More generally, the flexible gusset may be more flexible than the flap 12, e.g. the gusset may be made from a more flexible material than the flap 12.

In some embodiments the flap 12 may comprise a laminate material having two or more layers, and the flexible gusset may have fewer layers than the laminate material of the flap 12 such that the stiffness of the flexible gusset is lower than the stiffness of the flap.

This may present a particularly convenient way of providing a flexible gusset which is straightforward to manufacture.

For example, the laminate material of the flap 12 may comprise a fabric which is made up of two or more layers sandwiched together.

For example, the strap member may be made from a laminate material, the laminate material being used to manufacture at least the flexible gusset and the flap. In other words, the flap and the flexible gusset may be derived from the same starting material, e.g. the laminate. Accordingly, the flexible gusset may be provided by removing or omitting one or more layers of the laminate material at the desired location of the relief region 22 such that the relief region 22 has fewer layers than and is less stiff than the flap 12. For example, a cutout portion defining the relief region 22 may be provided in just one layer of the laminate, whilst the remaining layers of the laminate form the flexible gusset. Accordingly, there may be no need for additional joining processes to join the gusset to the flap 12 and the adjacent portion 24, since the remaining layers of the gusset will be integral to the flap 12 and the adjacent portion 24.

In some embodiments, at least one of the relief region 22 and a sensor location of the main strap 14 are visible to the subject when the sensor support strap 10 is being donned by the subject.

In this way, the relief region(s) 22 and/or the sensor location may be used as alignment feature(s) for enabling the sensor support strap 10 to be positioned correctly on the subject.

For example, a sensor of the sensor arrangement may be visible when supported by the sensor support strap 10. Alternatively, the sensor support strap 10 may include a marking to indicate the location of the sensor.

In a non-limiting illustrative example, such as that of FIG. 5, the sensor support strap 10 may be configured to support a sensor centrally on the subject's foot (see sensor location C of FIG. 5). The relief region 22 may also be visible, such as in FIG. 5. Accordingly, these features may be used to ensure that the sensor support strap 10 is aligned correctly on the subject's foot.

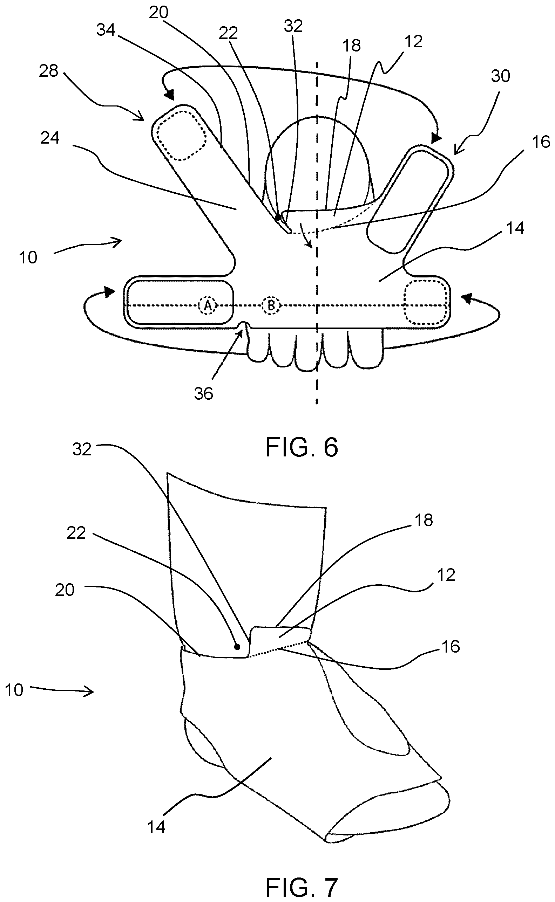

In FIG. 6, it is shown how the sensor support strap 10 may support more than one sensor, such as sensors at locations A and B. Sensor location B may be visible to the subject during use, since it is located on the top of the foot, so may be utilized as an alignment feature. In contrast, sensor location A is less likely to be visible to the subject, since it will likely be located on the underside of the subject's foot. The relief region 22 of FIG. 6 may also be visible, and accordingly used for alignment purposes.

More generally, the sensor support strap 10 may include any number of sensor locations and/or relief regions 22, which may or may not be visible. When said sensor locations and/or relief regions 22 are visible, they may be used for alignment of the sensor support strap 10.

The sensor support strap 10 may comprise sensor attachment means for supporting sensor(s) of the sensor arrangement at the sensor location(s), such as clip(s), slot(s), or pocket(s) into which the sensor(s) may be inserted. In some embodiments the attachment means may allow the sensor arrangement, e.g. the sensor(s), to be removed from the sensor support strap 10 by the subject, allowing the strap 10 to be washed, for example. Alternatively, the sensor arrangement may not be removeable from the strap 10, in other words, the sensors may be integral to the strap 10.

In some embodiments, the sensor support strap 10 comprises an elongate strap element 28 for wrapping at least partly around the body part, with a strap element relief region of the at least one relief region 22 separating part of the elongate strap element 28 from the flap 12. Thus, the strap 10 may be effectively secured to the body part whilst the comfort of the subject may be enhanced.

When the strap element relief region separates part of the elongate strap element 28 from the flap 12, it may be possible to provide a single flap 12, such as the flap 12 shown in FIG. 6. This may be an advantageous configuration of the sensor support strap 10 for use on certain body parts, such as body parts with less curvature.

In some embodiments, the sensor support strap 10 comprises a further elongate strap element 30 joinable to the elongate strap element 28, with the further elongate strap element 30 having a shorter length than the elongate strap element 28.

In such embodiments, the strap element relief region may be located closer to the elongate strap element 28 than the further elongate strap element 30, as shown in Fig. 6. By configuring the strap in this way, such that the strap element relief region is located closer to the longer strap 28 than the shorter strap 30, the stiffness of the shorter strap 30 may be increased. According, the ease of securing the strap 10 to the subject may be enhanced, since it may be easier to handle the shorter strap 30.

The further elongate strap element 30 and the elongate strap element 28 may be joinable to each other by any suitable joining means, such as via a hook and loop fastener.

Alternatively, the sensor support strap 10 may not comprise either the elongate strap element 28 or the further elongate strap element 30 and may instead comprise another suitable securing means for securing the strap 10 on the body part. For example, the sensor support strap 10 may be configured as a sock designed to be slipped over the subject's foot.

In some embodiments, such as that of FIG. 5, the sensor support strap 10 comprises a further flap 12' and a second hinging portion 26 for allowing hinging of the further flap 12' to enlarge the opening.

By providing a further flap 12', e.g. a second flap, the sensor support strap 10 may be able to more effectively accommodate movement and/or the shape of the subject, reducing discomfort for the user. This may be an advantageous configuration of the sensor support strap 10 for use on certain body parts, such as more curved body parts.

In some embodiments, such as that of FIG. 5, the further flap 12' defines the adjacent portion 24, the further flap 12' being separated from the flap 12 by one of said at least one relief region 12. In other words, two flaps, namely the flap 12 and the further flap 12', may be straightforwardly separated by one relief region 22. This may decrease manufacturing complexity of the sensor support strap 10.

In embodiments in which the sensor support strap 10 is configured for use on a foot, such as those of FIGs. 5 to 7, the relief region 22 and the sensor location, such as sensor locations A, B and C, may be alignable during use along a notional line extending from the subject's ankle towards one or more toes on the corresponding foot.

For example, as shown in FIG. 5, the sensor support strap 10 may be configured to support a sensor located centrally on the subject's foot at sensor location C. In this example, the relief region 22 may also be located centrally to the foot such that the sensor location C and the relief region 22 may be aligned along a notional line extending from the subject's ankle to the middle toe of the subject.

In another example, as shown in FIG. 6, the sensor support strap 10 may be configured to support a sensor at location B, which is offset from the center of the subject's foot. In this situation, the relief region 22 may also be offset from the center of the subject's foot such that these features may be aligned on a notional line extending from the side of the subject's ankle to the outside toe.

More generally, depending on the desired sensor location, it may be desirable to vary the location of the relief region 22 accordingly. That is, by positioning alignment features such as the sensor location(s) and the relief region 22 on a single axis, which in this case extends from the ankle, it may be easier to align the strap on a wider range of foot sizes. In contrast, if the sensor support strap includes multiple alignment features located on different axis, the subject may have difficulty in aligning all of the features, due to the size and shape of their foot.

In some embodiments, such as those of FIGs. 5 and 6, the sensor support strap 10 comprises a further edge, with the further edge comprising a notch 36 which is alignable with one or more toes of the foot during use. The notch 36 may be used for alignment of the sensor support strap 10 on the foot, as an alternative, or in addition to the relief region 22 and the sensor location(s) being used for alignment.

In at least some embodiments, the relief region 22, the notch 36 and a sensor location of the sensor support strap 10 may be aligned along a (further) notional line extending from the relief region 22 to the notch 36. This (further) notional line may be the same as or different from the above-mentioned notional line extending from the subject's ankle towards one or more toes on the foot.

In embodiments in which the sensor and the relief region 22 are located centrally on the subject's foot, such as that shown in FIG. 5, the notch 36 may also be positioned centrally to the subject's foot.

In embodiments in which the sensor and the relief region 22 are intended to be located laterally on the subject's foot, such as that of FIG. 6, the notch 36 may also be located laterally.

More generally, and as discussed in relation to the relief region 22 and sensor location(s) when these features are used for alignment of the sensor support strap 10, it may be beneficial that the notch 36, the relief region 22, and the sensor location(s) are aligned on a single axis, to ensure the features may be aligned on a wide range of foot sizes.

The present disclosure also relates to a sensor support strap system comprising a sensor support strap 10 of any of the examples described herein, and the sensor arrangement suitable for being supported by the sensor support strap 10.

The sensor arrangement may comprise one or more sensors for being supported by the sensor support strap 10. For example, the sensor arrangement may comprise one or more vital signs sensors, such as PPG sensors.

The sensor arrangement may further comprise a controller for controlling operation of the sensor arrangement. For example, the controller may be configured to control operation of one or more sensors to perform measurements using the sensors.

The controller may be configured to cause a transmitter, e.g. a transmitter of the sensor arrangement, to transmit data, e.g. measurement data, to an external device such as a smartphone.

The sensor arrangement may be integral to the sensor support strap 10 such that the sensor arrangement may be permanently coupled to the sensor support strap 10. Alternatively, the sensor arrangement may be removable, e.g. detachable, from the sensor support strap 10. For example, the sensor support strap 10 may be useable with various different sensor arrangements, which may be selectively attached and detached from the sensor support strap 10.

The sensor support strap 10 and the sensor arrangement of the system may be supplied together, e.g. as a kit. Alternatively, the sensor support strap 10 and the sensor arrangement of the system may be supplied separately.

The present disclosure further relates to use of the sensor support strap 10. The use comprises allowing a subject to wear the sensor arrangement supported by the strap 10. As explained above, the sensor support strap 10 comprises the strap member which is securable to the body part of the subject. The strap member comprises the flap 12, the main portion 14, the hinging portion 16 provided between the flap 12 and the main portion 14, and the edge 34. The edge 34 at least partly defines the opening of the strap member in which the body part is receivable when the strap member is secured to the body part of the subject such that the flap region 18 of the edge 34 is contactable by the body part. The flap 12 is at least partly delimited by the flap region 18 and a flap side edge 32, the flap side edge 32 extending from the flap region 18 to reach the hinging portion 16 so as to cause the strap member to preferentially hinge at the hinging portion 16.

In some embodiments, the use comprises allowing the subject to wear the sensor support strap 10 comprising the strap member, wherein the strap member comprises at least one relief region 22 separating the flap side edge 32 from an adjacent portion 24 of the strap member, with the at least one relief region 22 being configured to enlarge when the flap 12 preferentially hinges at the hinging portion 16.

In some embodiments, the use comprises allowing the subject to wear the sensor support strap 10 comprising the at least one relief region 22, wherein the at least one relief region 22 comprises a split in the strap member.

Alternatively or additionally, the at least one relief region 22 comprises a flexible gusset connecting the flap 12 to the adjacent portion 24.

More generally, the use of the sensor support strap 10 may relate to use of any of the embodiments of the sensor support strap 10 disclosed herein.

The body part may comprise a moveable joint of the subject. The edge may, for example, be contactable by a bending area of the moveable joint.

The sensor support strap 10 may be configured for use on a foot of the subject. That is, the body part may comprise at least a portion of the foot, with the portion comprising at least part of the ankle of the foot. In such embodiments, the bending area may comprise part of the ankle between the subject's instep and the subject's shin.

The subject may be an infant and the body part may be a foot. In other words the sensor support strap 10 may be configured for use on a foot of an infant, such as a baby's foot.

The present disclosure additionally relates to a method of manufacturing the sensor support strap 10. As explained above, the sensor support strap 10 comprises the strap member which is securable to a body part of the subject. The strap member comprises the flap 12, the main portion 14, the hinging portion 16 provided between the flap 12 and the main portion 14, and the edge 34. The edge 34 at least partly defines the opening of the strap member in which the body part is receivable when the strap member is secured to the body part of the subject such that the flap region 18 of the edge 34 is contactable by the body part. The flap 12 is at least partly delimited by the flap region 18 and the flap side edge 32. The method comprises providing the strap member with the hinging portion 16, wherein the strap member comprises the flap side edge 32 which extends from the flap region 18 to reach the hinging portion 16 so as to cause the strap member to preferentially hinge at the hinging portion 16.

The providing the strap member with the hinging portion 16 can be implemented in any suitable manner. In some embodiments, the providing the strap member with the hinging portion 16 comprises providing a line of weakness, extending along a line defining a boundary between the flap 12 from the main portion 14, at which the strap member preferentially folds.

The line of weakness can be formed, for example, by compressing the strap member, e.g. by folding the flap 12 relative to the main portion 14, along the line defining the boundary between the flap 12 and the main portion 14.

In other embodiments, providing the hinging portion 16 comprises providing a strip of material to separate the flap 12 from the main portion 14, which strip of material is more flexible than the flap 12 and the main portion 14 so that the strip of material flexes, rather than the flap 12 and the main portion 14, during hinging of the strap member at the hinging portion 16.

In some embodiments, the method further comprises providing the at least one relief region 22, wherein the at least one relief region 22 separates the flap side edge 32 from an adjacent portion 24 of the strap member, the at least one relief region 22 being configured to enlarge when the flap 12 preferentially hinges at the hinging portion 16.

In some embodiments, providing the strap member with at least one relief region 22 comprises providing a split in the strap member.

Alternatively or additionally, providing the strap member with at least one relief region 22 comprises providing a flexible gusset connecting the flap 12 to the adjacent portion 24.

More generally, the method may comprise providing the sensor support strap 10, e.g. the strap member, of any of the embodiments of the sensor support strap 10 disclosed herein.

The body part may comprise a moveable joint of the subject. The edge may, for example, be contactable by a bending area of the moveable joint.

The sensor support strap may be configured for use on a foot of the subject. That is, the body part may comprise at least a portion of the foot, with the portion comprising at least part of the ankle of the foot. In such embodiments, the bending area may comprise part of the ankle between the subject's instep and the subject's shin.

The subject may be an infant and the body part may be a foot. In other words the sensor support strap may be configured for use on a foot of an infant, such as a baby's foot.

The subject may be an infant, such that the sensor support strap is configured for use on a foot of an infant, such as a baby's foot.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A sensor support strap (10) for allowing a subject to wear a sensor arrangement supported by the strap, the sensor support strap comprising:
a strap member securable to a body part of the subject, the strap member comprising:
a flap (12);
a main portion (14);
a hinging portion (16) provided between the flap and the main portion; and
an edge (34) which at least partly defines an opening of the strap member in which the body part is receivable when the strap member is secured to the body part of the subject such that a flap region (18) of the edge is contactable by the body part, wherein the flap is at least partly delimited by the flap region and a flap side edge (32), the flap side edge extending from the flap region to reach the hinging portion so as to cause the strap member to preferentially hinge at the hinging portion.

2. The sensor support strap (10) of claim 1, wherein the strap member comprises at least one relief region (22) separating the flap side edge (32) from an adjacent portion (24) of the strap member, the at least one relief region being configured to enlarge when the flap (12) preferentially hinges at the hinging portion (16).

3. The sensor support strap (10) of claim 2, wherein the at least one relief region (22) comprises a split in the strap member.

4. The sensor support strap (10) of claim 2 or 3, wherein the at least one relief region (22) comprises a flexible gusset connecting the flap (12) to the adjacent portion (24).

5. The sensor support strap (10) of claim 4, wherein the flap (12) comprises a laminate having two or more layers, the flexible gusset having fewer layers than the laminate such that the stiffness of the flexible gusset is lower than the stiffness of the flap.

6. The sensor support strap (10) of any of claims 2 to 5, wherein at least one of the relief region(s) (22) and a sensor location of the main strap (14) are visible to the subject when the sensor support strap is being donned by the subject.

7. The sensor support strap (10) of any of claims 2 to 6, comprising an elongate strap element (28) for wrapping at least partly around the body part, a strap element relief region of the at least one relief region (22) separating part of the elongate strap element from the flap (12).

8. The sensor support strap (10) of claim 7, comprising a further elongate strap element (30) joinable to the elongate strap element (28), the further elongate strap element having a shorter length than the elongate strap element.

9. The sensor support strap (10) of any of claims 1 to 8, comprising a further flap (12') and second hinging portion (26) for allowing hinging of the further flap to enlarge the opening.

10. The sensor support strap (10) of claim 9 when dependent from any of claims 2 to 6, wherein the further flap (12') defines the adjacent portion (24), the further flap being separated from the flap (12) by one of said at least one relief region (22).

11. The sensor support strap (10) of any of claims 1 to 10, wherein the sensor support strap is configured for use on a foot of the subject.

12. The sensor support strap (10) of claim 11, wherein the sensor support strap comprises a further edge, the further edge comprising a notch (36) which is alignable with one or more toes of the foot during use.

13. A sensor support strap system comprising:
a sensor support strap (10) of any of claims 1 to 12; and
a sensor arrangement for being supported by the strap.

14. Use of a sensor support strap (10) for allowing a subject to wear a sensor arrangement supported by the strap, the sensor support strap comprising:
a strap member securable to a body part of the subject, the strap member comprising:
a flap (12);
a main portion (14); and
a hinging portion (16) provided between the flap and the main portion; and
an edge (34) which at least partly defines an opening of the strap member in which the body part is receivable when the strap member is secured to the body part of the subject such that a flap region (18) of the edge is contactable by the body part, wherein the flap is at least partly delimited by the flap region and a flap side edge (32), the flap side edge extending from the flap region to reach the hinging portion so as to cause the strap member to preferentially hinge at the hinging portion.

15. A method of manufacturing a sensor support strap (10) for allowing a subject to wear a sensor arrangement supported by the strap, the sensor support strap comprising:
a strap member securable to a body part of the subject, the strap member comprising:
a flap (12);
a main portion (14);
a hinging portion (16) provided between the flap and the main portion; and an edge (34) which at least partly defines an opening of the strap member in which the body part is receivable when the strap member is secured to the body part of the subject such that a flap region (18) of the edge is contactable by the body part, wherein the flap is at least partly delimited by the flap region and a flap side edge (32), wherein the method comprises providing the strap member with a hinging portion, the strap member comprising a flap side edge extending from the flap region to reach the hinging portion so as to cause the strap member to preferentially hinge at the hinging portion.
